# EUROPÄISCHE PATENTSCHRIFT

(11) **EP 2 928 968 B1**
(45) Veröffentlichungstag und Bekanntmachung des Hinweises auf die Patenterteilung: **08.03.2017**
(21) Anmeldenummer: 13802525.9
(22) Anmeldetag: 29.11.2013
(51) Int. Cl.: C09C 1/50, F23D 14/22, F23D 99/00, B01J 19/24, F23D 5/18, G01N 33/00

(54) **RUSSGENERATOR**
SOOT GENERATOR
GÉNÉRATEUR DE SUIE

(30) Priorität: 07.12.2012 CH 27352012
(43) Veröffentlichungstag der Anmeldung: 14.10.2015
(73) Patentinhaber: Jing, Lianpeng, 3052 Zollikofen (CH)
(72) Erfinder: JING, Lianpeng, 3052 Zollikofen (CH)
(74) Vertreter: AMMANN PATENTANWÄLTE AG BERN
(86) Internationale Anmeldenummer: PCT/CH2013/000204
(87) Internationale Veröffentlichungsnummer: WO 2014/085941

(56) Entgegenhaltungen:
- EP-A1- 1 055 877
- DE-A1- 2 842 977
- Dr. Lutz v. Meyerinck: "Druckkocher: Funktion, Aufbau, Betrieb und Reparatur", Ohlson 38 , November 2011 (2011-11), Seiten 1-24, XP002693940, Gefunden im Internet: URL:http://www.ohlson38.de/Ohlson38%20Docu mnets/Ohlson38%20Manuals/files/petroleum.V 207.pdf [gefunden am 2012-03-14]

## Beschreibung

Die Erfindung bezieht sich auf einen Russgenerator gemäss Oberbegriff des Anspruchs 1.

In den Patenten EP 1 055 877 B1 und EP 1 590 408 B1 des gleichen Anmelders sind Russgeneratoren beschrieben, mit welchen Russpartikel mit wohldefinierten chemischen und physikalischen Eigenschaften erzeugbar sind. Dazu werden Brennstoff und Oxidationsgas derart in eine Brennkammer geführt, dass eine Russpartikel erzeugende Diffusionsflamme gebildet wird. Die Spitze der Diffusionsflamme wird mit einem Löschgas angeströmt, sodass die Verbrennungsprozesse gestoppt und die Russpartikel weggeleitet werden. Diese Russgeneratoren haben sich besonders bewährt beim Einsatz von gasförmigen Brennstoffen. Es hat sich gezeigt, dass flüssige Brennstoffe weniger gut geeignet sind, Russpartikel mit den gewünschten Eigenschaften zu erzeugen In der Patentanmeldung DE 28 42 977 A1 wird ein Russgenerator beschrieben, bei dem flüssige Brennstoffe vergast werden. In dem Artikel L. Mevering, "Druckkocher: Funktion, Aufbau, Betrieb und Reparatur" (Quelle: URL:http://www.ohlson38.de/ Ohlson38%20Documnets/Ohlson38%20Manuals/files/petroleum.V207.pdf), wird eine Vorrichtung zum Vorwärmen flüssiger Brennstoffe beschrieben.

Es ist daher eine Aufgabe der vorliegenden Erfindung, die bekannten Russgeneratoren so weiterzuentwickeln, dass besonders bei Verwendung von flüssigem Brennstoff Russpartikel mit möglichst wohldefinierten Eigenschaften herstellbar sind.

Ein erfindungsgemässer Russgenerator, welcher diese Aufgabe löst, ist im Anspruch 1 angegeben. In den weiteren Ansprüchen sind bevorzugte Ausführungen sowie eine Verwendung des Russgenerators angegeben.

Gemäss Anspruch 1 umfasst der erfindungsgemässe Russgenerator eine Erwärmungseinrichtung zum Erwärmen der Brennstoffzufuhrleitung an mindestens einer Erwärmungsstelle. Dies erlaubt es, flüssigen Brennstoff vor der eigentlichen Verbrennung so zu erwärmen, dass er zumindest teilweise verdampft. Dies verbessert die Verbrennung und begünstigt somit die Erzeugung von Russpartikeln mit wohldefinierten Eigenschaften.

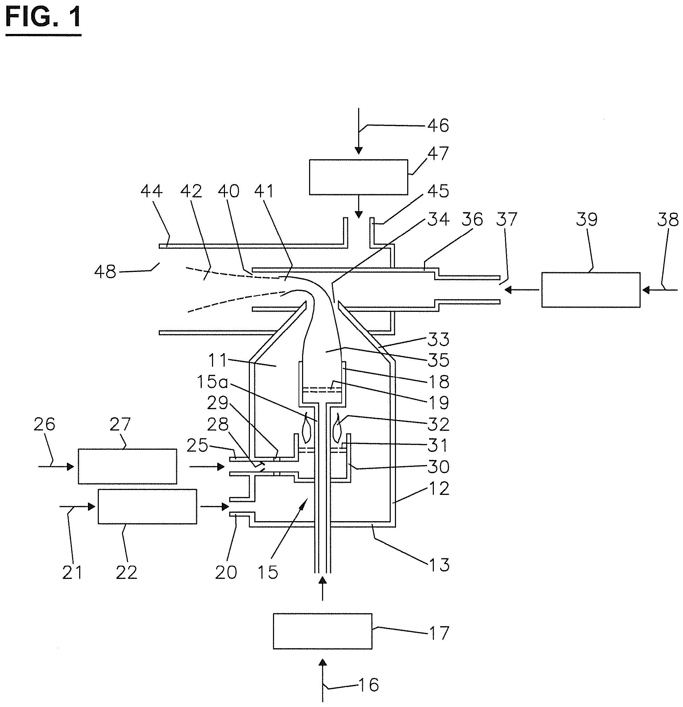

Die Erfindung wird im Folgenden anhand eines Ausführungsbeispiels erläutert unter Bezugnahme auf
Fig. 1, welche schematisch einen erfindungsgemässen Russgenerator im Querschnitt zeigt.

Der in Fig. 1 dargestellte Russgenerator umfasst eine Brennkammer 11, welche seitlich durch eine Aussenwand 12 begrenzt ist. Die Aussenwand 12, die z. B. zylindrisch ausgebildet ist, weist einen Boden 13 auf, durch welchen hindurch eine Zufuhrleitung in Form eines Innenrohrs 15 verläuft. Dieses weist z. B. eine zylindrische Form auf und ist vorzugsweise koaxial zur Aussenwand 12 angeordnet.

Der Einlass des Innenrohrs 15 ist mit einem (nicht dargestellten) Brennstoffbehälter verbindbar zur Zufuhr von Brennstoff 16. Zur Regelung der Zuflussrate (Liter pro Sekunde) ist zwischen dem Brennstoffbehälter und dem Einlass des Innenrohrs 15 eine geeignete Einstelleinrichtung 17, z. B. ein Flussregler oder eine Dosierpumpe vorgesehen. Im Brennstoffbehälter ist flüssiger Brennstoff gespeichert, z. B. Dieseltreibstoff, Benzin, Kerosin, Hexan, etc.

Das Innenrohr 15 mündet in einem Auslass 18. Dieser ist oben offen ausgebildet und ist durch eine Erweiterung im Rohrquerschnitt gebildet. Der Querschnitt der Öffnung des Auslasses 18 ist somit grösser als der Querschnitt des Endes 15a des Innenrohrs 15. Der Auslass 18 ist z. B. durch einen zylindrischen Rohrabschnitt mit einem Boden gebildet, in welchem sich der Einlass des Innenrohrs 15 befindet. Der Auslass 18, der in Fig. 1 stufenförmig dargestellt ist, kann auch anders geformt sein, z. B. in Form einer kontinuierlichen Erweiterung, die an der Innenfläche sowie an der Aussenfläche stufenlos ist. Im Auslass 18 ist optional eine Blende 19 mit Durchgangsöffnungen angeordnet.

In der Aussenwand 12 mündet an ihrem gemäss Fig. 1 unteren Ende eine zweite Zufuhrleitung 20, über welche Oxidationsmittel 21 in die Brennkammer 11 leitbar ist. Die zweite Zufuhrleitung 20 ist über einen Flussregler 22 zur Regulation der Zuflussrate mit einem (nicht dargestellten) Behälter verbindbar, in welchem das Oxidationsmittel 21 gespeichert ist. Als Oxidationsmittel 21 sind beispielsweise Pressluft, synthetische Luft (80 % Stickstoffgas, 20 % Sauerstoffgas) oder andere Oxidationsgase denkbar.

Weiter weist der Russgenerator eine nachfolgend genauer erläuterte Erwärmungseinrichtung 25-31 auf, die zum Erwärmen der ersten Zufuhrleitung 15 an mindestens einer Stelle 15a dient. Eine dritte Zufuhrleitung 25 zur Zufuhr von Brennstoff 26 führt durch die Aussenwand 12 hindurch und mündet in einem Vorwärmbrenner 30. Die dritte Zufuhrleitung 25 ist über einen Flussregler 27 zur Regulation der Zuflussrate mit einem (nicht dargestellten) Behälter verbindbar, in welchem der Brennstoff 26 gespeichert ist. Dieser ist vorzugsweise ein Brenngas, z. B. kohlenwasserstoffhaltiges Gas wie Propan.

Der Abschnitt der dritten Zufuhrleitung 25, welcher innerhalb der Aussenwand 12 verläuft, ist mit einer oder mehreren Eingangsöffnungen 29 versehen. Über diese gelangt im Betrieb Oxidationsmittel 21, welches über die zweite Zufuhrleitung 20 in die Brennkammer 11 eingespeist wird, in die dritte Zufuhrleitung 25, wo es mit dem Brennstoff 26 vermischt wird. Um das Ansaugen des Oxidationsmittels 21 zu begünstigen, enthält die dritte Zufuhrleitung 25 stromaufwärts der Eingangsöffnungen 29 eine Verengung 28, beispielsweise eine Düse, wodurch der Venturi-Effekt nutzbar ist. Dabei erzeugt im Betrieb der aufgrund der Verengung 28 schneller strömende Brennstoff 26 einen Unterdruck, so dass Oxidationsmittel 21 durch die Eingangsöffnungen 29 angesaugt und mit dem Brennstoff 26 vermischt wird.

Der Vorwärmbrenner 30 verläuft um das Innenrohr 15 herum und ist hier auslassseitig offen ausgestaltet. Im Vorwärmbrenner 30 ist optional eine Blende 31 mit Durchgangsöffnungen angeordnet. Das Ende 15a des Innenrohrs 15, welches durch den Vorwärmbrenner 30 hindurchverläuft, bildet eine Erwärmungsstelle, an welcher der Brennstoff 16 im Innenrohr 15 erwärmbar ist. Zur Erzeugung einer Erwärmungsstelle ist der Vorwärmbrenner 30 nicht unbedingt erforderlich. Es ist auch möglich, diesen wegzulassen, so dass am Auslass der Zuleitung 25 eine Flamme erzeugbar ist, die quer zur Zufuhrleitung 15 gerichtet ist.

Zumindest der zu erwärmende Teil 15a des Innenrohrs 15 ist aus einem thermisch leitenden Material gefertigt, so dass an der Aussenwandung erzeugte Hitze in das Innere des Innenrohrs 15 leitbar ist.

Nachfolgend ist der Teil des Russgenerators erläutert, der zur eigentlichen Erzeugung und Abfuhr der Russpartikel dient.

Die Brennkammer 11 weist auslassseitig eine Wandung 33 auf, die sich in Strömungsrichtung gesehen verjüngt und endseitig mit einer Öffnung 34 versehen ist, welche in einer Russwegführleitung 36 angeordnet ist. Diese umfasst einlassseitig einen Einlass 37 zur Zufuhr von Löschgas 38, welches z. B. in einem (nicht dargestellten) Löschgasbehälter in Form einer Gasflasche gespeichert ist. Zur Regulation der Zuflussrate des Löschgases 38 in die Russwegführleitung 36 ist zwischen dem Löschgasbehälter und dem Einlass 37 ein Flussregler 39 angebracht.

Am anderen Ende der Russwegführleitung 36 befindet sich ein Auslass 40, aus welchem u.a. das Löschgas zusammen mit den Russpartikeln hinausströmen kann. Die Russwegführleitung 36 ist z. B. durch ein Rohr oder mehrere, zusammengefügte Rohrabschnitte gebildet.

Vorzugsweise ist zum Zünden des Brennstoffes 16 und/oder des Brenngases 26 mindestens ein (nicht dargestelltes) Zündmittel in der Brennkammer 11 vorgesehen, welches beispielsweise eingerichtet ist, Zündfunken zu erzeugen.

Der Russgenerator wird wie folgt betrieben:

Zuerst wird im Vorwärmbrenner 30 oder - falls weggelassen - auslassseitig der Zuleitung 25 eine Flamme 32 zum Erwärmen der ersten Zufuhrleitung 15 erzeugt, indem Brenngas 26 in die dritte Zufuhrleitung 25 geleitet, dort mit Oxidationsmittel 21 vermischt und schliesslich gezündet wird. Ist die Blende 31 vorgesehen, so ist es möglich, die Strömungsverhältnisse möglichst laminar zu halten und daher eine besonders ruhig brennende Flamme 32 zu erzeugen. Diese erwärmt die erste Zufuhrleitung 15 an der Erwärmungsstelle 15a. Wird nun flüssiger Brennstoff 16 in die Zufuhrleitung 15 geleitet, so verdampft er bei der Erwärmungsstelle 15a und gelangt überwiegend in gasförmiger Gestalt in den Auslass 18, wo er schliesslich gezündet wird. Die Zündung kann z. B. einfacher Weise derart erfolgen, dass kurzzeitig mehr Brenngas 26 zum Vorwärmbrenner 30 geleitet wird, so dass die grösser werdende Flamme 32 bis zur auslassseitigen Öffnung des Auslasses 18 reicht und den dort ausströmenden Brennstoff 16 zündet.

Nach der Zündung bildet sich über der Öffnung des Auslasses 18 eine Diffusionsflamme 35 aus. Dabei wird der ausströmende Brennstoff 16 von dem durch die Zufuhrleitung 20 eingespeisten Oxidationsmittel 21 in einer im Wesentlichen parallelen Strömung umhüllt. Durch das Vorsehen der Blende 19 im Auslass 18 können die Strömungsverhältnisse im ausströmenden Brennstoff 16 in verbessertem Masse laminar gehalten werden. Es bildet sich dann eine im Wesentlichen zylinderförmige Diffusionsflamme 35 aus, in welcher durch Verbrennung des Brennstoffs 16 mit dem Oxidationsmittel 21 Russpartikel gebildet werden.

Je nach Anwendungszweck der Russpartikel ist es zu deren Herstellung jedoch auch denkbar, den Russgenerator so zu gestalten, dass sich eine turbulente Flamme über dem Auslass 18 ausbildet.

Zum Erhalt der Russpartikel wird Löschgas 38 durch die Russwegführleitung 36 geleitet, sodass dieses mit der aus der Brennkammer 11 austretenden Stoffströmung, welche u.a. die Russpartikel enthält, vermischt wird und so die Verbrennungsprozesse bei der Löschstelle 41 gestoppt werden. Die Russpartikel werden dann durch die Löschgasströmung zum Auslass 40 fortgetragen.

Als Löschgas ist ein Inertgas einsetzbar wie Stickstoffgas, Edelgas oder Kohlendioxid. Es ist auch denkbar, Luft als Löschgas zu verwenden, insbesondere Luft, die kühler als die Flamme 35 ist. Beispielsweise kann Luft aus der Umgebung mittels Pumpe durch die Russwegführleitung 36 geleitet werden. Da die Lufttemperatur in etwa der Raumtemperatur entspricht und somit niedriger als die Flammentemperatur ist, werden aufgrund einer Kühlung die Verbrennungsprozesse bei der Löschstelle 41 gestoppt. Dieser Vorgang kann durch eine relativ hohe Strömungsgeschwindigkeit der Luft in der Russwegführleitung 36 unterstützt werden, wodurch das aus der Öffnung 34 ragende Ende der Flamme 35 förmlich ausgeblasen wird.

Optional weist der Russgenerator Verdünnungsmittel 44-48 auf. Zu diesem Zweck ist ein Mantelohr 44 vorgesehen, in welchem der Auslass 40 der Russwegführleitung 36 angeordnet und welches z. B. zylindrisch ausgebildet ist. Stromaufwärts vom Auslass 40 befindet sich ein Anschluss 45 zum Einspeisen eines Verdünnungsgases 46 (beispielsweise Luft) in das Mantelrohr 44. Stromabwärts vom Auslass 40 befindet sich das offene Ende 48 des Mantelrohrs 44.

Im Betrieb strömt das Verdünnungsgas 46 in das Mantelrohr 44 und vermischt sich mit der aus dem Auslass 40 tretenden gelöschten Stoffströmung 42, so dass diese verdünnt wird. Alternativ zur Verwendung eines Mantelrohrs 44 ist es auch denkbar, endseitig der Russwegführleitung 36 Öffnungen vorzusehen, z. B. zwei symmetrisch angeordnete Öffnungen, durch welche hindurch das Verdünnungsgas 46 zur Stoffströmung 42 hin leitbar ist.

Durch Vorsehen eines Flussreglers 47 ist die Zuflussrate des Verdünnungsgases 46 einstellbar, wodurch entsprechend die Konzentration der Russpartikel (Partikelanzahl pro m³), welche schliesslich aus dem offenen Ende des Russgenerators herausströmen, geändert werden kann.

Der hier beschriebene Russgenerator eignet sich besonders zur Herstellung von Russpartikeln aus flüssigem Brennstoff. Je nach Anwendungszweck ist es aber auch möglich, einen gasförmigen Brennstoff einzusetzen, um die Flamme 35 zu erzeugen. In diesem Fall wird die Vorwärmflamme 32, wenn überhaupt, nur zeitweise eingesetzt, z. B. zum Zünden der Flamme 35. Der Russgenerator kann somit wahlweise mit flüssigem oder gasförmigen Brennstoff verwendet werden.

Mit dem Russgenerator können Russpartikel mit einer im Wesentlichen reproduzierbaren Grössenverteilung und definierten chemischen Zusammensetzung hergestellt werden.

Verschiedene Parameter beeinflussen die Russpartikel-Eigenschaften wie Grösse und Konzentration sowie chemische Zusammensetzung:
- Art und Zusammensetzung des Brennstoffs
- Zuflussrate (in l/sec) des Brennstoffs
- Zuflussrate des Oxidationsmittels
- Zuflussrate des Löschgases
- Löschstelle der Diffusionsflamme durch Löschgas (im Wesentlichen gegeben durch den Abstand zwischen der Öffnung des Auslasses 18 und der Einmündung 34 der Brennkammer 11)
- etc.

Je nach Wahl der Parameter können z. B. Russpartikel erzeugt werden, deren Grösse auf einer logarithmischen Skala im Wesentlichen gaussverteilt ist, wobei das Maximum kleiner als 1 Mikrometer ist, z. B. im Bereich von 0.01 bis 0.3 Mikrometer. Es ist aber auch möglich, durch entsprechende Wahl des Brennstoffs und/oder Gestaltung der Russwegführleitung, Russpartikel herzustellen, die grösser als 1 Mikrometer sind.

Der Russgenerator ist vielseitig einsetzbar, z. B. zur Eichung von Russpartikel-Messgeräten, zur Abgasmessung bei Motorfahrzeugen, zum Prüfen von Filtern, zur Untersuchung von Aerosolen, zum Testen von Rauchmeldern, zur Herstellung von Russproben und Referenzmaterial, etc.

Aus der vorangehenden Beschreibung sind dem Fachmann zahlreiche Abwandlungen zugänglich, ohne den Schutzbereich der Erfindung zu verlassen, der durch die Ansprüche definiert ist.

So ist es z. B. möglich, den Russgenerator derart zu gestalten, dass die Zufuhrleitung 15 ausserhalb der Brennkammer 11 erwärmbar ist. Dazu werden beispielsweise der Vorwärmbrenner 30 - falls vorhanden - und die Zufuhrleitung 25 auf der anderen Seite des Bodens 13 angeordnet.

Die Öffnungen 28 in der dritten Zufuhrleitung 25 können auch ausserhalb der Brennkammer 11 angeordnet sein, um Oxidationsmittel anzusaugen, welches auch Umgebungsluft sein kann.

Weiter ist es denkbar, die Erwärmungseinrichtung 25-31 so zu gestalten, dass die Zufuhrleitung 15 an mehreren Stellen erwärmbar ist.

Es ist auch möglich, die Erwärmungseinrichtung so auszugestalten, dass anstelle einer vorgemischten Flamme auch eine andere Art von Flamme im Vorwärmbrenner 30 erzeugbar ist, z. B. eine Diffusionsflamme. Ist ein Vormischen von Oxidationsmittel nicht vorgesehen, so können die Öffnungen 28 und die Verengung 29 entfallen.

Weiter ist die Erwärmungseinrichtung auch so ausgestaltbar, dass die Zufuhrleitung 15 elektrisch erwärmbar ist, indem z. B. eine oder mehrere elektrische Heizelemente vorgesehen werden.

Als weitere Ausführungsform ist der Russgenerator auch so ausgestaltbar, dass derselbe Brennstoff zur Bildung der Flammen 32 und 33 verwendet wird. Da die Flamme 32 lediglich zum Vorwärmen der Zufuhrleitung 15 benötigt wird, ist eine optimale Verbrennung hier nicht unbedingt erforderlich. Es ist daher möglich, auch flüssigen Brennstoff zur Bildung beider Flammen 32 und 33 zu verwenden.

Die Brennkammer 11 mit dem Auslass 34 braucht nicht zwingendermassen quer zur Russwegführleitung 36 angeordnet zu sein, wie dies in Fig. 1 gezeigt ist. Es ist denkbar, das obere Ende 33 der Aussenwand 12 gebogen auszugestalten, sodass der mittlere Teil der Flamme 33 entsprechend umgelenkt werden kann. Je nach Anwendungszweck kann der Winkel, um welchen das obere Ende 33 gebogen ist, von 0 bis 90 Grad gewählt sein.

Weiter ist es denkbar, in der Russwegführleitung eine Verengung vorzusehen, um aufgrund des Venturi-Effektes bei der Öffnung 34 einen Unterdruck zu erzeugen und dadurch Oxidationsmittel anzusaugen, wie dies im Patent EP 1 590 408 B1 beschrieben ist. In einer besonders einfachen Ausführungsform kann dieser Unterdruck dazu verwendet werden, Umgebungsluft als Oxidationsmittel durch eine nach aussen reichende Öffnung in der Brennkammer 11 anzusaugen. In diesem Fall können die Gasflasche mit dem darin gespeichertem Oxidationsmittel 21 sowie die Zufuhrleitung 20 und der Flussregler 22 weggelassen werden.

Der Russgenerator kann auch so gestaltet sein, dass mehrere Russpartikel erzeugende Flammen vorgesehen sind.

## Patentansprüche

1. Russgenerator mit
einer Brennkammer (11), in welcher Brennstoff (16) mit einem Oxidationsmittel (21) in mindestens einer Russpartikel erzeugenden Flamme (33) verbrennbar ist,
einer Brennstoffzufuhrleitung (15) zur Zufuhr von Brennstoff (16) in die Brennkammer (11) und
einer mit der Brennkammer (11) verbundenen Russwegführleitung (36), in welche Löschgas (38) leitbar und aus welcher Russpartikel wegführbar sind, **gekennzeichnet durch**
eine Erwärmungseinrichtung (25-31) zum Erwärmen der Brennstoffzufuhrleitung (15) an mindestens einer Erwärmungsstelle (15a).

2. Russgenerator nach Anspruch 1, wobei die Erwärmungseinrichtung (25-31) eine Brennstelle (30) zur Erzeugung einer Erwärmungsflamme (32) umfasst, wobei vorzugsweise die Brennstelle (30) um die Brennstoffzufuhrleitung (15) oder quer dazu verläuft.

3. Russgenerator nach Anspruch 2, wobei die Brennstelle durch einen Auslass (30) gebildet ist, in welchem vorzugsweise eine Blende (31) mit Durchgangsöffnungen angeordnet ist.

4. Russgenerator nach einem der vorangehenden Ansprüche, wobei die Erwärmungseinrichtung (25-31) eine weitere Brennstoffzufuhrleitung (25) aufweist zur Zufuhr von Brennstoff (26), welcher vorzugsweise verschieden ist vom Brennstoff (16) in der ersten Brennstoffzufuhrleitung (15) und/oder welcher vorzugsweise ein Brenngas ist.

5. Russgenerator nach Anspruch 4, wobei die weitere Brennstoffzufuhrleitung (25) mindestens eine Öffnung (29) aufweist, durch welche Oxidationsmittel (21) zuführbar ist zur Vermischung mit dem in der weiteren Brennstoffzufuhrleitung (25) geleiteten Brennstoff (26).

6. Russgenerator nach Anspruch 5, wobei in der weiteren Brennstoffzufuhrleitung (25) eine Verengung (28) stromaufwärts von der mindestens einen Öffnung (29) angeordnet ist.

7. Russgenerator nach einem der vorangehenden Ansprüche, bei welchem in der Brennkammer (11) mindestens ein Zündmittel angeordnet ist.

8. Russgenerator nach einem der vorangehenden Ansprüche, wobei die erste Brennstoffzufuhrleitung (15) in einem Auslass (18) mündet, welcher vorzugsweise eine sich aufweitende Form aufweist und/oder in welchem vorzugsweise eine Blende (19) mit Durchgangsöffnungen angeordnet ist.

9. Russgenerator nach einem der vorangehenden Ansprüche, bei welchem die Brennkammer (11) quer zur Russwegführleitung (36) angeordnet ist, sodass die Flamme (33) quer mit Löschgas anströmbar ist.

10. Russgenerator nach einem der vorangehenden Ansprüche, welcher eine Verdünnungseinrichtung (44-48) aufweist zum Verdünnen der Konzentration der aus der Russwegführleitung (36) weggeführten Russpartikel.

11. Verwendung des Russgenerators nach einem der vorangehenden Ansprüche zur Erzeugung von Russpartikeln, welche insbesondere als Russprobe und/oder Referenzmaterial dienen und/oder zum Eichen von Messgeräten, zum Testen von Sensoren und/oder zum Prüfen von Filtern verwendet werden.

## Claims

1. Soot generator, comprising
a combustion chamber (11), in which fuel (16) can be burnt with an oxidizing agent (21) in at least one flame (33) that produces soot particles,
a fuel feed line (15) for supplying fuel (16) to the combustion chamber (11) and
a soot removal line (36), which is connected to the combustion chamber (11) and into which quenching gas (38) can be conducted and out of which soot particles can be removed, **characterized by**
a heating device (25-31) for heating the fuel feed line (15) at at least one heating location (15a).

2. Soot generator according to claim 1, wherein the heating device (25-31) comprises a combustion site (30) for creating a heating flame (32), wherein the combustion site (30) preferably runs around the fuel feed line (15) or transversely thereto.

3. Soot generator according to claim 2, wherein the combustion site is formed by an outlet (30), in which an aperture (31) having through-openings is preferably situated.

4. Soot generator according to any one of the preceding claims, wherein the heating device (25-31) has an additional fuel feed line (25) for supplying fuel (26), which is preferably different from the fuel (16) in the first fuel feed line (15) and/or which is preferably a combustible gas.

5. Soot generator according to claim 4, wherein the additional fuel feed line (25) has at least one opening (29) through which oxidizing agent (21) can be supplied for mixing with the fuel (26) directed into the additional fuel feed line (25).

6. Soot generator according to claim 5, wherein a constriction (28) is provided in the additional fuel feed line (25) upstream from the at least one opening (29).

7. Soot generator according to any one of the preceding claims, wherein at least one igniting means is arranged in the fuel chamber (11).

8. Soot generator according to any one of the preceding claims, wherein the first fuel feed line (15) opens into an outlet (18), which preferably has a widened shape and/or in which an aperture (19) with through-openings is preferably arranged.

9. Soot generator according to any one of the preceding claims, wherein the fuel chamber (11) is arranged transversely to the soot removal line (36), so that the flame (33) can be exposed transversely to an oncoming flow of quenching gas.

10. Soot generator according to any one of the preceding claims, which has a diluting device (44-48) for diluting the concentration of the soot particles discharged out of the soot removal line (36).

11. Use of a soot generator according to any one of the preceding claims, for producing soot particles, which serve in particular as a soot sample and/or as a reference material and/or which are used for calibrating measurement devices, for testing sensors and/or for testing filters.

## Revendications

1. Générateur de suie comprenant
une chambre de combustion (11) dans laquelle un combustible (16) peut être brûlé avec un oxydant (21) dans au moins une flamme (33) produisant des particules de suie,
une conduite d'alimentation (15) en combustible pour l'alimentation d'un combustible (16) à la chambre de combustion (11) et
une conduite de dégagement (36) de suie reliée à la chambre de combustion (11), à laquelle un gaz d'extinction (38) peut être alimenté et par laquelle des particules de suie peuvent être dégagés, **caractérisé par**
un dispositif de réchauffement (25-31) pour réchauffer la conduite d'alimentation en combustible (15) en au moins un point de réchauffement.

2. Générateur de suie selon la revendication 1, où le dispositif de réchauffement (25-31) comprend un point de combustion (30) afin de produire une flamme de réchauffement (32), le point de combustion s'étendant préférablement autour de la conduite d'alimentation en combustible (15) ou transversalement à celle-ci.

3. Générateur de suie selon la revendication 2, où le point de combustion est formé par un orifice de sortie (30) dans lequel est préférablement disposé un diaphragme (31) présentant des ouvertures de passage.

4. Générateur de suie selon l'une des revendications précédentes, où le dispositif de réchauffement (25-31) comprend une conduite d'alimentation en combustible (25) supplémentaire pour l'alimentation d'un combustible (26) qui est préférablement différent du combustible (16) dans la première conduite d'alimentation en combustible (15) et/ou qui est préférablement un gaz combustible.

5. Générateur de suie selon la revendication 4, où ladite conduite d'alimentation en combustible (25) supplémentaire présente au moins une ouverture (29) par laquelle un oxydant (21) peut être alimenté pour être mélangé au combustible (26) amené dans ladite conduite d'alimentation en combustible (25) supplémentaire.

6. Générateur de suie selon la revendication 5, où dans ladite conduite d'alimentation en combustible (25) supplémentaire est agencé un rétrécissement (28) en amont de ladite au moins une ouverture (29).

7. Générateur de suie selon l'une des revendications précédentes, où dans la chambre de combustion (11) est agencé au moins un moyen d'allumage.

8. Générateur de suie selon l'une des revendications précédentes, où ladite première conduite d'alimentation en combustible (15) débouche dans un orifice de sortie (18) qui présente préférablement une forme évasée et/ou dans lequel est préférablement agencé un diaphragme (19) présentant des ouvertures de passage.

9. Générateur de suie selon l'une des revendications précédentes, où la chambre de combustion (11) est agencée transversalement à la conduite de dégagement de suie (36), permettant de faire affluer ledit gaz d'extinction sur la flamme (33) en direction transversale.

10. Générateur de suie selon l'une des revendications précédentes, lequel comprend un dispositif de dilution (44-48) afin de diluer la concentration des particules de suie dégagé par la conduite de dégagement de suie (36).

11. Utilisation du générateur de suie selon l'une des revendications précédentes pour générer des particules de suie qui servent particulièrement d'échantillons de suie et/ou de matériau de référence et/ou pour l'étalonnage d'appareils de mesure, pour tester des capteurs et/ou pour examiner des filtres.
